# EUROPEAN PATENT SPECIFICATION

(11) **EP 1 177 010 B1**
(45) Date of publication and mention of the grant of the patent: **23.11.2005**
(21) Application number: 00925259.4
(22) Date of filing: 04.05.2000
(51) Int. Cl.: A61N 1/05

(54) **TEMPORARY HEART LEAD AND CORRESPONDING EXTERNAL CONNECTION DEVICE**
ZEITWEILIGE HERZLEITUNG UND ENTSPRECHENDE ÄUSSERLICHE ANSCHLUSSVORRICHTUNG
DERIVATION CARDIAQUE TEMPORAIRE ET DISPOSITIF DE CONNEXION EXTERNE CORRESPONDANT

(30) Priority: 07.05.1999 DE 19922275
(43) Date of publication of application: 06.02.2002
(73) Proprietor: Ethicon GmbH, 22851 Norderstedt (DE)
(72) Inventor: WALTHER, Christoph, D-24568 Kattendorf (DE); BLUME, Stefan, D-79285 Ebringen (DE)
(74) Representative: UEXKÜLL & STOLBERG
(86) International application number: PCT/EP2000/004001
(87) International publication number: WO 2000/067835

(56) References cited:
- WO-A-93/09840
- WO-A-97/47351
- US-A- 4 442 840
- US-A- 4 693 258

## Description

The invention relates to a system with a temporary heart lead and an external connection device for same.

A temporary heart lead has a distal end-region with at least one distal electrode and a proximal end-region with at least one proximal electrode, a respective distal electrode being connected in electrically conductive manner to a respective proximal electrode. During a surgical operation the distal end-region of a temporary heart lead is fastened to the heart wall, for example with the help of a surgical needle provided in the distal end-region of the temporary heart lead, which needle can later be severed. The lead is then guided through the thorax to the outside. A surgical needle which is fastened to the proximal end of the lead is used for this. The at least one proximal electrode can be connected to an external pacemaker or else an ECG monitor in order to stimulate or monitor the patient's heart activity if need be.

Plugs for connection to the stripped end of a heart electrode wire, which can be connected in each case to a proximal electrode of a temporary heart lead, are known from DE 34 45 102 C1 and DE 44 37 818 A1. Two such plugs must be used with a bipolar temporary heart lead. It is also known to break off the surgical needle, which is attached in the proximal end-region of a temporary heart lead and connected in electrically conductive manner to a distal electrode of the heart lead, at a set fracture point and insert the residual piece of the surgical needle directly as a plug into a socket at the external pacemaker. The previously known plugs are however relatively awkward to handle; in the last-mentioned case, the absence of electrical insulation of the residual piece of the surgical needle is also problematical.

A system with a temporary heart lead and an external connection device, in which the temporary heart lead is a bipolar line with a distal end-region having two distal electrodes and a proximal end-region having two proximal electrodes, is described in EP 0 613 388 B1. A surgical needle with a set fracture point is fastened in the proximal end-region, the part of the needle that remains after the breaking-off of the needle at the heart lead representing one of the two proximal electrodes. The external connection device has a housing with an entry aperture and a needle exit aperture. The proximal end-region of the heart lead can be introduced into the housing through the entry aperture with the help of the surgical needle and advanced until the needle projects out of the needle exit aperture. Located in the inside of the housing are two electric contacts, one of which engages at the said residual piece of the needle and the other at the other proximal electrode of the heart lead when the needle has reached its set position. In this position, the set fracture point of the needle lies in the vicinity of the needle exit aperture, so that the part of the needle which includes the needle tip can be broken off. The two electric contacts of the connection device are connected to a cable which is set up for connection to an external pacemaker.

Although the external connection device disclosed in EP 0 613 388 B1 simplifies the connection of a temporary heart lead to an external pacemaker, it requires a temporary heart lead with a surgical needle which is relatively expensively constructed because of the set fracture point. Furthermore, the threading of the heart lead into the connection device with the help of the surgical needle requires some skill, as the needle tip must be guided through the needle exit aperture which is essentially matched to the diameter of the needle.

The object of the invention is to create a system with a temporary heart lead and an external connection device for same (as well as a connection device adapted to same) which basically requires a less expensively constructed surgical needle in the proximal end-region of the temporary heart lead and which is secure in application, easy to handle and cheap to produce.

This object is achieved by a system with a temporary heart lead and an external connection device for same having the features of Claim 1. Claim 16 relates to the external connection device matched to the system. Advantageous configurations of the invention result from the dependent claims.

The system according to the invention has a temporary heart lead and an external connection device for same. The temporary heart lead has a distal end-region with at least one distal electrode and a proximal end-region with at least one proximal electrode and a surgical needle that can be severed. This surgical needle is not set up as an electrode. A respective distal electrode is connected in electrically conductive manner to a respective proximal electrode. The connection device has a housing with an entry aperture and a needle exit aperture and a needle-severing device. Arranged in the inside of the housing are rapid-action contacts, assigned to respective proximal electrodes of the temporary heart lead, which can be connected via at least one cable to an external pacemaker (or for example to an external monitoring apparatus).

Since neither the surgical needle in the proximal end-region of the temporary heart lead nor a residual piece of this needle is set up as a proximal electrode, the surgical needle as a whole can be subjected to a surface treatment which improves its service properties (for example blackening, siliconization) but reduces its electrical conductivity. Furthermore, it is possible in principle, when connecting the temporary heart lead to the external connection device, to remove the surgical needle completely with the help of the needle-severing device. In particular, the surgical needle need not have a set fracture point. The system according to the invention is easy to handle and secure and can be produced cheaply. The connection device can be set up as a reusable article and should be sterilizable in this case.

According to the invention, the needle exit aperture has a slit with a first end and a second end, which slit has an opening at its first end and passes there into the entry aperture. The housing of the connection device preferably has a base, a side wall and an upper part, the entry aperture being formed in the side wall and the slit in the base or the upper part. With such a configuration of the connection device, the proximal end-region of the temporary heart lead can be attached particularly quickly, easily and securely to the connection device. To this end, the proximal end-region of the heart lead can be gripped at the surgical needle, which is preferably straight, and introduced into the entry aperture, the surgical needle being aligned essentially perpendicular to the slit and preferably essentially perpendicular to the base or to the upper part of the housing. As the slit is open at its first end and passes there into the entry aperture, the needle projects with its end-region containing the needle tip from the needle exit aperture and can thus be advanced without problems as far as the second end of the slit. In the process, the following region of the temporary heart lead is pulled through the entry aperture into the inside of the housing until the proximal electrode or the proximal electrodes engage the assigned rapid-action contacts. An electric contact is thereby immediately created.

Various configurations are conceivable for the needle-severing device.

In a preferred version the surgical needle has an end-piece, arranged opposite its tip, which can be broken off at a set fracture point, and the needle-severing device has a clamping device, arranged in the region of the second end of the slit, into which the end-piece of the surgical needle can be clamped in an arrangement of the surgical needle running transversely to the slit. With this version, the surgical needle is pushed in, in the previously explained manner, as far as the second end of the slit until the end-piece of the needle locks in the clamping device. It is held fast by this so that the residual part of the surgical needle with the needle tip which projects from the needle exit aperture can be broken off without problems. The clamped-in end-piece of the surgical needle does not serve as a proximal electrode.

In another configuration the needle-severing device has a cutting zone which is accessible via an opening arranged in the region of the second end of the slit. In this case the surgical needle need not have a set fracture point. After the introduction of the proximal end-region of the temporary heart lead into the connection device in the manner described above, the point of attachment of the surgical needle to the rest of the temporary heart lead is located in the region of the cutting zone. There it is accessible through the opening, so that the needle can be severed without problems from the rest of the temporary heart lead with the help of a cutting instrument such as for example a knife.

It is also conceivable for the needle-severing device to have a cutting device which is arranged for example in the inside of the housing of the connection device and can be actuated via an outward-guided operating element.

In a preferred version the housing has a winding device for taking up surplus line length of the heart lead. The winding device can have at least two indentations provided on the outside of the housing. With such a configuration the surplus line length of the heart lead, i.e. the region projecting from the patient's thorax, can be wound up on the winding device. The connection device can then be fixed to the patient's skin for example with the help of adhesive strips.

Many different configurations are conceivable for the rapid-action contact or contacts. One version, in which a contact spring is provided, is described in more detail below.

The connection device preferably has a tension relief means for the proximal end-region of the temporary heart lead. The tension relief means can for example have a leaf spring flexing in one direction and locking in the opposite direction which is set up to engage at the proximal end-region of the temporary heart lead. With a tension relief means configured in this way, the proximal end-region of the temporary heart lead can be introduced into the connection device without the tension relief means having to be released in order to do so. However, if an attempt is made to move in the reverse direction the tension relief means locks immediately; an additional arresting measure is not necessary.

The heart lead can be configured as a monopolar heart lead with one distal and one proximal electrode. In a preferred version, the heart lead is configured as a bipolar heart lead with two distal and two proximal electrodes. In the latter case the heart lead is preferably coaxial at least in its proximal end-region; but it can also be coaxial over its entire length and have e.g. the form of a coaxial cable. If the heart lead is coaxially formed at least in its proximal end-region, the two proximal electrodes are preferably annular and spaced to each other. The annular electrodes preferably have the same outer diameter, their outer diameter being matched to the outer diameter of the outer insulation in the proximal region of the heart lead. This outer insulation runs e.g. over the greatest part of the length of the temporary heart lead and is preferably also located in the region between the two annular electrodes. With such a configuration, there are no projecting parts in the proximal end-region of the temporary heart lead and no recesses, so that the proximal end-region of the heart lead is essentially smooth and can be easily guided through the thorax with the help of the surgical needle.

The distal end-region of the temporary heart lead can be configured in any known way, for example with anchors for rapid fastening to the heart tissue or with a (preferably bent) surgical needle.

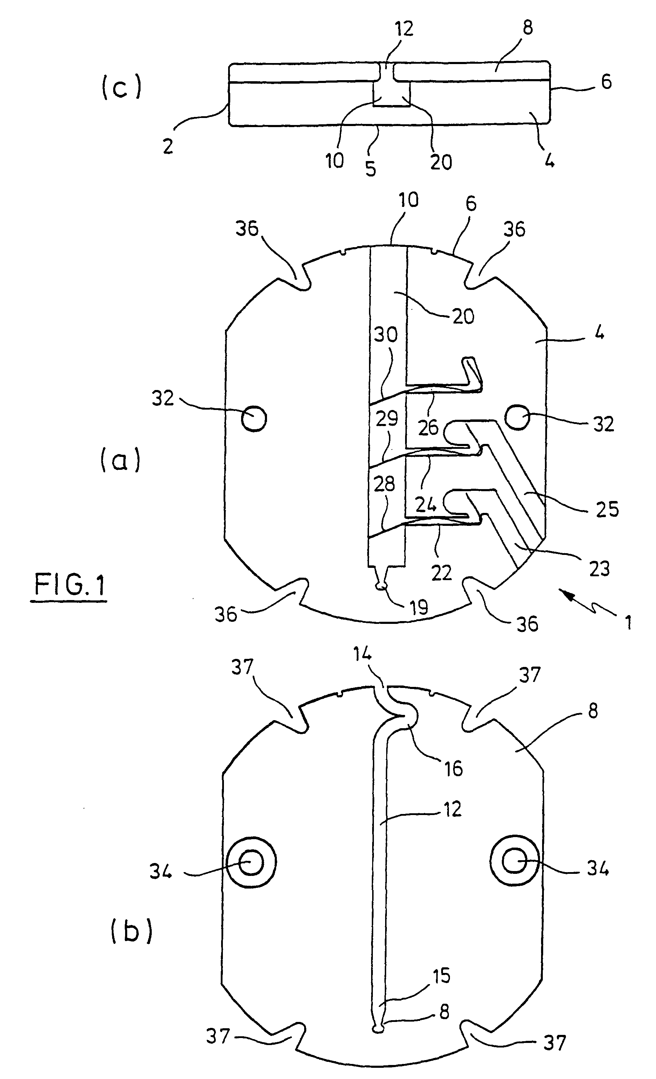

The invention is explained in more detail below with reference to embodiments. The figures show in
- Figure 1: in part (a) a plan view of the lower part of the housing of the connection device in a first version of the system according to the invention, the upper part of the housing being removed, in part (b) a plan view of the matching upper part of the housing and in part (c) an end view of the assembled connection device looking in the direction of the arrow from part (a),

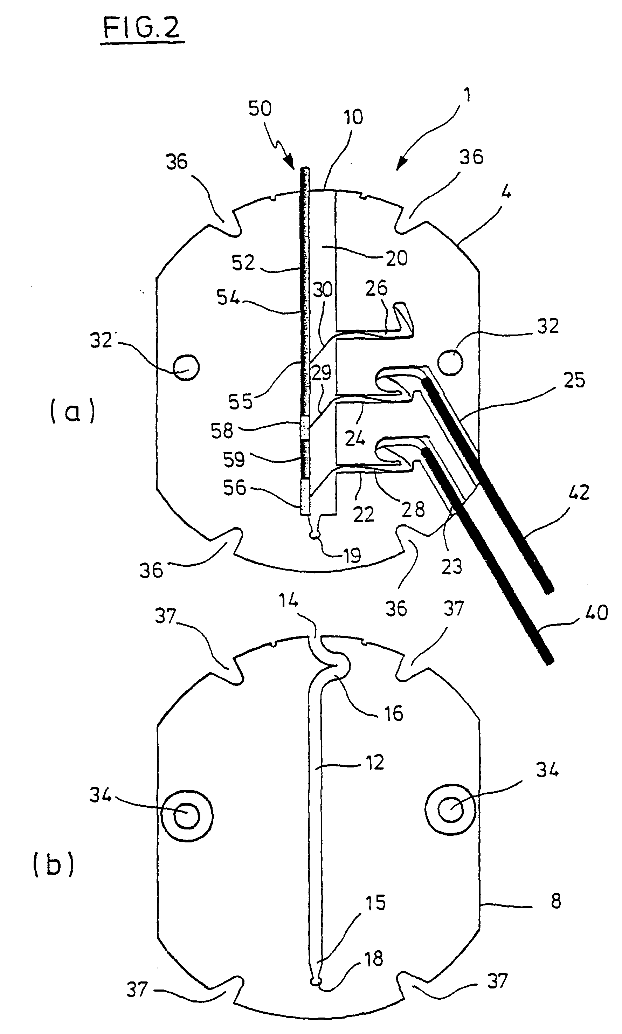

- Figure 2: in part (a) a plan view of the lower part of the housing according to Figure 1(a), two cables being fitted as supply lines to an external pacemaker and the proximal end-region of a temporary heart lead being inserted, and in part (b) a plan view of the upper part of the housing according to Figure 1(b),

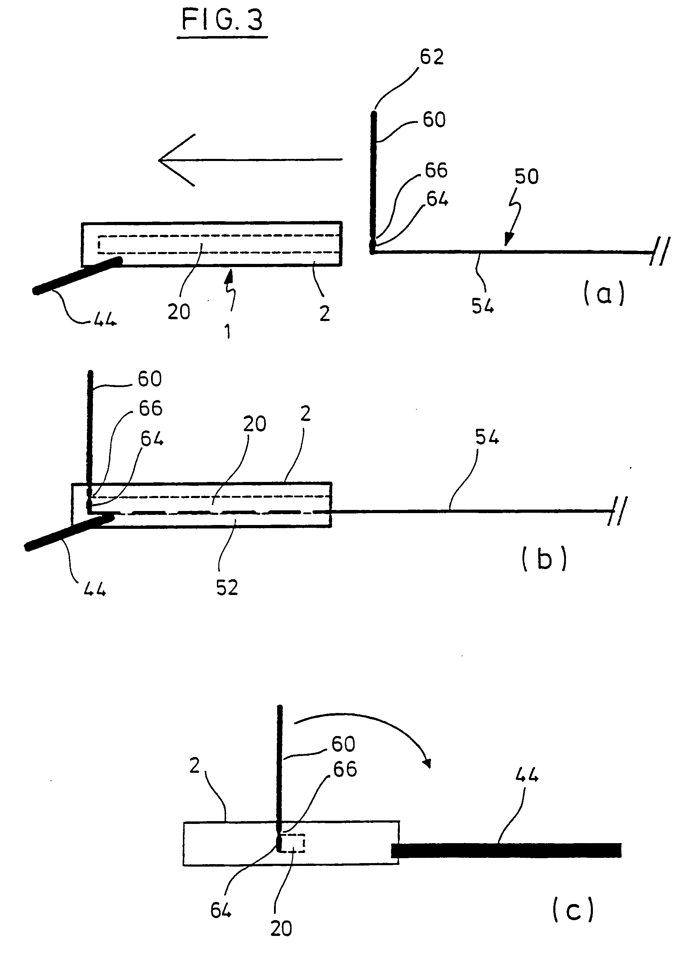

- Figure 3: a schematic representation of successive steps for inserting the proximal end-region of the temporary heart lead into the connection device (part (a) and part (b)) and for severing the surgical needle (part (c)) in the system according to Figures 1 and 2,

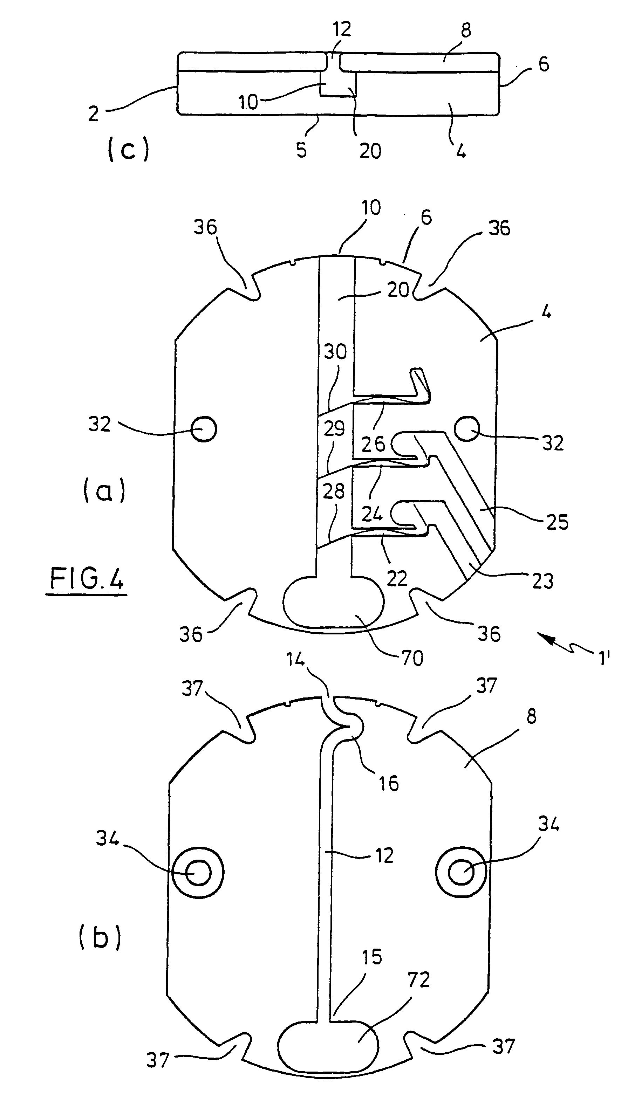

- Figure 4: in part (a) a plan view of the lower part of the housing of the connection device in a second version of the system according to the invention, the upper part of the housing being removed, in part (b) a plan view of the matching upper part of the housing and in part (c) an end-view of the assembled connection device looking in the direction of the arrow from part (a), and

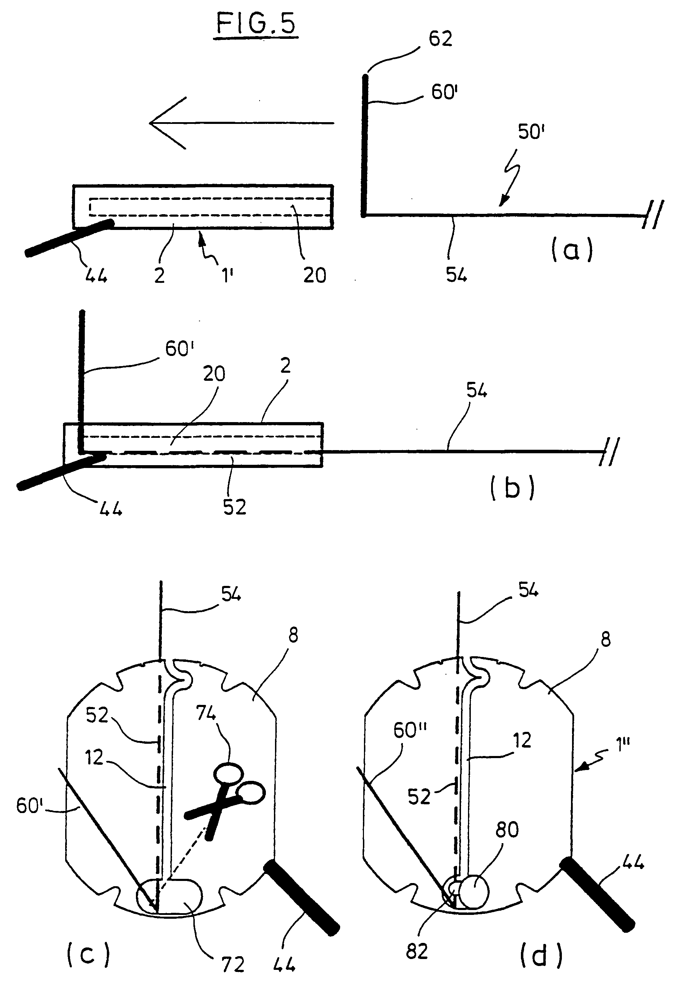

- Figure 5: a schematic representation of successive steps for inserting the temporary heart lead into the connection device (part (a) and part (b)) and for severing the surgical needle (part c)) in the system according to Figure 4 and in part(d) a schematic representation of the step for severing the surgical needle in a further version of the system according to the invention.

There are represented in Figure 1 essential parts of an external connection device 1 of a first version of a system which includes a temporary heart lead and this connection device 1. The connection device 1 has a housing 2 which in the embodiment consists of a lower part 4 with a base 5 and with a side wall 6 and of an upper part 8. The lower part 4 and the upper part 8 are each designed in one piece as an injection moulding, made in the embodiment from polycarbonate, a sterilizable and auotclavable plastics material. Figure 1(a) shows a plan view of the lower part 4, whose side wall 6 is very thick in places, and Figure 1(b) a plan view of the upper part 8. In the assembled state the lower part 4 and the upper part 8 lie one above the other, see Figure 1(c), which shows a view from the side looking in the direction according to the arrow drawn in in Figure 1(a).

An entry aperture 10 is developed in the lower part 4 on the face of the housing 2 visible in Figure 1 (c). The upper part 8 is provided with a slit 12, running straight over wide sections, with a first end 14 and a second end 15, which is open at its first end 14 and passes there into the entry aperture 10, see Figure 1 (c). In the vicinity of its first end 14 the slit 12 has a section designed as a chicane 16, see Figure 1(b).

Located at the second end 15 of the slit 12 is a constriction 18 which broadens out into an opening essentially bounded in the form of an arc, see Figure 1(b). A correspondingly shaped recess 19 (referred to hereinafter as constriction for the sake of simplicity), which lies exactly below the constriction 18 in the assembled state of the housing 2, is also provided in the lower part 4, see Figure 1(a). This recess does not however extend through the base 5 of the lower part 4. The constrictions 18 and 19 form a clamping device, the function of which is explained further below.

Extending in longitudinal direction of the lower part 4 between the entry aperture 10 and the constriction 19 is a duct 20 which is designed as a recess running essentially in a straight line, as represented in Figures 1(a) and 1(c). In the assembled state of the housing 2, the duct 20 runs beneath the slit 12, but has a greater width than the slit 12, see Figure 1(c).

Extending laterally from the duct 20 are a first channel 22, which passes into a first connection duct 23 extending as far as the outside of the housing 2, a second channel 24 which passes into a second connection duct 25 extending as far as the outside of the housing 2, and a further channel 26, as represented in Figure 1(a). A first contact spring 28 is inserted into the first channel 22, a second contact spring 29 into the second channel 24 and a leaf spring 30 into the further channel 26. The contact springs 28 and 29 and the leaf spring 30 are of similar construction. They each have a free end, projecting into the duct 20, which in each case extends as far as the opposite duct wall and is somewhat angled towards the constriction 19. The opposite end-regions of the contact springs 28 and 29 and of the leaf spring 30 are strongly angled and prevent a slippage of these springs whose positions are moreover fixed by the base 5 of the lower part 4 and the upper part 8. The mode of operation of the contact springs 28 and 29 and of the leaf spring 30 is further described below with reference to Figure 2.

The upper part 8 is secured to the lower part 4 of the housing 2 by two tapped holes 32 in the lower part 4, into which screws inserted through two sunk bores 34 in the upper part 8 are inserted.

The lower part 4 has four indentations 36 which are aligned relative to corresponding indentations 37 in the upper part 8. In the assembled state of the housing 2 these indentations form a winding device for taking up surplus line length of the temporary heart lead (see below).

Figure 2 shows how a first cable 40 and a second cable 42 respectively are inserted into the first connection duct 23 and the second connection duct 25, which cables each have stripped ends. These bare cable ends are in electrical contact with the first contact spring 28 and the second contact spring 29 respectively. The cables 40 and 42, which are shown as separate cables in Figure 2(a), are combined to form a joint line 44 (see Figure 3). Located at the non-represented end of the joint line 44 is a plug for connecting the connection device 1 to an external pacemaker or to an external monitoring device.

In the embodiment, the cables 40 and 42 and the joint line 44 are a constituent of the connection device 1. However, it is also conceivable to provide this connection line to an external pacemaker or to an external monitoring device as a separate component. The contact springs 28 and 29 or corresponding components can be configured according to requirements. Conceivable, for example, are easily detachable connections or additional tension relief means.

Figure 2(a) shows how a temporary heart lead 50 is inserted with its proximal end-region 52 into the duct 20. The distal end-region of the temporary heart lead 50 is not represented in the Figures. In the embodiment, it contains two distal electrodes which are connected in electrically conductive manner via a line 54, which has an outer insulation 55, to a first proximal electrode 56 and a second proximal electrode 58. In the embodiment, the line 54 is designed as a coaxial cable practically over its whole length with an inner conductor and an outer conductor. The inner conductor is wire-shaped and guided through as far as the first proximal electrode 56. The outer conductor is annular and extends as far as the second proximal electrode 58. An annular insulation is located between the inner conductor and the outer conductor. The first proximal electrode 56 and the second proximal electrode 58 are each designed as annular sheaths whose outer diameter is as great as the outer diameter of the outer insulation 55 of the line 54. Also located between the first proximal electrode 56 and the second proximal electrode 58 is an insulation 59 whose outer diameter is likewise matched to that of the outer insulation 55. The proximal end-region 52 of the temporary heart lead 50 therefore has no noteworthy projections or depressions, which greatly facilitates the introduction into the connection device 1.

Figure 3 illustrates in schematic manner how the temporary heart lead 50 is connected to the connection device 1 and how a surgical needle 60, which is located at the end of the temporary heart lead 50, is then severed.

The surgical needle 60 is configured as a straight thorax needle and has a tip 62. In this version, the surgical needle 60 has, opposite its tip 62, an end-piece 64 which is connected to the remainder of the needle via a set fracture point 66.

After the distal end-region of the temporary heart lead 50 has been attached in the patient's heart region, the line 54 is guided through the patient' s thorax with the help of the surgical needle 60. In order to connect the temporary heart lead 50 to the connection device 1, the surgical needle 60 is first bent to an angle of ca. 90°, so that it assumes the position shown in Figure 3(a). The proximal end-region 52 of the temporary heart lead 50 can then be pushed through the entry aperture 10 in the direction of the arrow from Figure 3(a) into the duct 20. The surgical needle 60 protrudes through the slit 12. With the help of the surgical needle 60, the proximal end-region 52 of the temporary heart lead 50 can be pulled without problems through the duct 20. For this, it is first necessary for the chicane 16 to be overcome in the vicinity of the entry aperture 10, but this then effectively prevents the line 54 from emerging upwards out of the slit 12. In the further course of the movement in the direction of the arrow from Figure 3(a), the leaf spring 30, the second contact spring 29 and the first contact spring 28 are successively bent in by the proximal end-region 52, but lie against the proximal end-region 52. The movement is continued until the end-piece 64 of the surgical needle 60 locks in the clamping device 18, 19. This situation is represented in Figure 3(b).

Figure 3 (c) is a view looking towards the face of the housing 2 lying opposite the entry aperture 10 and illustrates how the surgical needle 60 can be broken off without problems at the set fracture point 66 by moving it in the direction of the curved arrow.

It is thus easily and quickly possible to attach the temporary heart lead 50 to the connection device 1. The surgical needle 60 is removed without problems and securely. A reliable electrical contact is produced, via the contact springs 28 and 29 and the proximal electrodes 56 and 58, between the distal electrodes of the temporary heart lead 50 and the joint line 44 which can be attached for example to an external heart pacemaker. If an attempt is made to pull the line 54 out of the duct 20, the friction between the free end of the leaf spring 30 and the outer insulation 55 of the line 54 causes the leaf spring 30 to be tensioned even more firmly onto the insulation 55. In this way the proximal end-region 52 is securely clamped between the leaf spring 30 and the wall of the duct 20. As the slit 12 is narrower than the width of the duct 20 and because of the chicane 16 the proximal end-region 52 of the temporary heart lead 50 cannot emerge upwards out of the duct 20 either.

The end-piece of the line 54 projecting from the patient's thorax can advantageously be wound onto the housing 2, so that the indentations 36 and 37 prevent it from slipping off again.

Figure 4 shows a second version of the system with a temporary heart lead 50' (see Figure 5) and an external connection device 1' for same. The first and second versions differ in respect of the needle-severing device. Parts and components which are the same in both versions are provided in Figure 4 (and in Figure 5) with the reference numbers used in Figures 1 to 3 and therefore do not need to be explained.

As Figure 4(a) shows, in the second version there is located at the end of duct 20 lying opposite the entry aperture 10 a recess 70, configured as a cutting zone, which is delimited at the bottom by the base 5 of the lower part 4. The recess 70 is accessible via an opening 72 in the upper part 8 which has the same outline shape as the recess 70.

In the second version of the system, the surgical needle, here numbered 60', has no set fracture point. In order to introduce the proximal end 52 of the temporary heart lead 50', the procedure is initially as in the first version, see Figure 5(a) and Figure 5(b). For this, the surgical needle 60' which is angled vis-à-vis the line 54 is guided through as far as the end of the recess 70, as is schematically represented in Figure 5(c), the inclined course of the surgical needle 60' indicating that the surgical needle 60' projects out from the plane of the paper. When this situation is reached, the surgical needle 60' can be cut off with the help of scissors 74 which are introduced into the recess 70. The recess 70 thus serves as a cutting zone or guide zone which makes possible a rapid and secure severance of the surgical needle 60' and minimizes a risk of injury.

A variant of the second version of the system is represented in Figure 5(d). In it, the cutting zone of the second version is somewhat differently configured and contains a cutting device 80 with a fixed blade 82. In order to sever the surgical needle, which is numbered 60" here, after the introduction of the proximal end-region 52 of the temporary heart lead into the connection device 1", the end of the line is guided along the fixed blade 82 in the region of the attachment point of the surgical needle 60".

In the embodiments, the temporary heart lead is designed as a bipolar coaxial cable. Other versions, for example as a bipolar double litz wire, as a monopolar line or else with more than two distal or proximal electrodes (e.g. with four distal and four proximal electrodes) are also conceivable. The number of rapid-action contacts assigned to the proximal electrodes of the temporary heart lead is preferably matched to the number of proximal electrodes.

## Claims

1. System with a temporary heart lead (50) and an external connection device (1) for same,
- in which the temporary heart lead (50) has a distal end-region with at least one distal electrode and a proximal end-region (52) with at least one proximal electrode (56, 58) and a severable surgical needle (60), not set up as an electrode, a respective distal electrode being connected in electrically conductive manner to a respective proximal electrode (56, 58), and
- in which the connection device (1) has a housing (2) with an entry aperture (10) and a needle exit aperture (12) and has a needle-severing device (18, 19; 70; 80), there being arranged in the inside of the housing (2) rapid-action contacts (28, 29), assigned to respective proximal electrodes (56, 58) of the temporary heart lead (50), which can be connected via at least one cable (40, 42) to an external pacemaker
**characterised in that** the needle exit aperture is formed as a slit (12) with a first end (14) and a second end (15) in a wall (8) of the housing (2), which slit has an opening at its first end (14) and there passes into the entry aperture (10) and which slit runs alongside of a duct (20) formed in the inside of the housing (2) for accomodating part of the proximal end-region (52) of the temporary heart lead (50) and communicates with said duct (20).

2. System according to claim 1, **characterized in that** the housing (2) has a base (5), a side wall (6) and an upper part (8), the entry aperture (10) being formed in the side wall (6) and the slit (12) in the base (5) or in the upper part (8).

3. System according to claim 1 or 2, **characterized in that** the surgical needle (60) has an end-piece (64), arranged opposite its tip (62), which can be broken off at a set fracture point (66), and **in that** the needle-severing device (18, 19) has a clamping device (18, 19), arranged in the region of the second end (15) of the slit (12), into which the end-piece (64) of the surgical needle (60) can be clamped in an arrangement of the surgical needle (60) running transversely to the slit (12).

4. System according to claim 1 or 2, **characterized in that** the needle-severing device (70) has a cutting zone (70) which is accessible via an opening (72) arranged in the region of the second end (15) of the slit (12).

5. System according to claim 1 or 2, **characterized in that** the needle-severing device (80) has a cutting device (80, 82).

6. System according to one of claims 1 to 5, **characterized in that** the housing (2) has a winding device (36, 37) for taking up surplus line length of the heart lead.

7. System according to claim 6, **characterized in that** the winding device (36, 37) has at least two indentations (36, 37) provided on the outside of the housing (2).

8. System according to one of claims 1 to 7, **characterized in that** at least one rapid-action contact (28, 29) has a contact spring (28, 29).

9. System according to one of claims 1 to 8, **characterized in that** the connection device (1) has a tension relief means (30) for the proximal end-region (52) of the temporary heart lead (50).

10. System according to claim 9, **characterized in that** the tension relief means (30) has a leaf spring (30), flexing in one direction and locking in the opposite direction, which is set up to engage at the proximal end-region (52) of the temporary heart lead (50).

11. System according to one of claims 1 to 10, **characterized in that** the heart lead is configured as a monopolar heart lead with one distal and one proximal electrode.

12. System according to one of claims 1 to 10, **characterized in that** the heart lead (50) is configured as a bipolar heart lead with two distal and two proximal electrodes (56, 58).

13. System according to claim 12, **characterized in that** the heart lead (50) is coaxial at least in its proximal end-region (52).

14. System according to claim 13, **characterized in that** the two proximal electrodes (56, 58) are annular and are spaced to each other.

15. System according to claim 14, **characterized in that** the two annular electrodes (56, 58) have the same outer diameter, which is matched to the outer diameter of the outer insulation (55) in the proximal region of the heart lead (50).

16. External connection device (1) for a temporary heart lead (50), which has a distal end-region with at least one distal electrode and a proximal end-region (52) with at least one proximal electrode (56, 58) and a severable surgical needle (60), not set up as an electrode, a respective distal electrode being connected in electrically conductive manner to a respective proximal electrode (56, 58),
in which the connection device (1) has a housing (2) with an entry aperture (10) and a needle exit aperture (12) and a needle-severing device (18, 19; 70; 80), there being arranged in the inside of the housing (2) rapid-action contacts (28, 29), assigned to respective proximal electrodes (56, 58) of the temporary heart lead (50), which can be connected via at least one cable (40, 42) to an external pacemaker,
**characterised in that** the needle exit aperture is formed as a slit (12) with a first end (14) and a second end (15) in a wall (8) of the housing (2), which slit has an opening at its first end (14) and there passes into the entry aperture (10) and which slit runs alongside of a duct (20) formed in the inside of the housing (2) for accomodating part of the proximal end-region (52) of the temporary heart lead (50) and communicates with said duct (20).

17. Connection device according to claim 16, **characterized by** the features of one of claims 2 to 15 relating to the connection device (1).

## Patentansprüche

1. System mit einer temporären Herzleitung (50) und einer externen Verbindungsvorrichtung (1) dafür,
- wobei die temporäre Herzleitung (50) einen distalen Endbereich mit mindestens einer distalen Elektrode und einen proximalen Endbereich (52) mit mindestens einer proximalen Elektrode (56, 58) und einer abtrennbaren, nicht als Elektrode eingerichteten chirurgischen Nadel (60) aufweist, wobei eine jeweilige distale Elektrode elektrisch leitend mit einer jeweiligen proximalen Elektrode (56, 58) verbunden ist, und
- wobei die Verbindungsvorrichtung (1) ein Gehäuse (2) mit einer Eintrittsöffnung (10) und einer Nadelaustrittsöffnung (12) und eine Nadelabtrenneinrichtung (18, 19; 70; 80) aufweist, wobei im Innenraum des Gehäuses (2) jeweiligen proximalen Elektroden (56, 58) der temporären Herzleitung (50) zugeordnete Schnellkontakte (28, 29) angeordnet sind, die über mindestens ein Kabel (40, 42) mit einem externen Herzschrittmacher verbindbar sind,
**dadurch gekennzeichnet, daß** die Nadelaustrittsöffnung als Schlitz (12) mit einem ersten Ende (14) und einem zweiten Ende (15) in einer Wand (8) des Gehäuses (2) geformt ist, der an seinem ersten Ende (14) eine Öffnung hat und dort in die Eintrittsöffnung (10) übergeht und der entlang eines im Innenraum des Gehäuses (2) ausgebildeten Kanals (20) zum Aufnehmen eines Teils des proximalen Endbereichs (52) der temporären Herzleitung (50) verläuft und mit dem Kanal (20) in Verbindung steht.

2. System nach Anspruch 1, **dadurch gekennzeichnet, daß** das Gehäuse (2) einen Boden (5), eine Seitenwand (6) und ein Oberteil (8) hat, wobei die Eintrittsöffnung (10) in der Seitenwand (6) und der Schlitz (12) in dem Boden (5) oder dem Oberteil (8) ausgebildet ist.

3. System nach Anspruch 1 oder 2, **dadurch gekennzeichnet, daß** die chirurgische Nadel (60) ein gegenüber ihrer Spitze (62) angeordnetes Endstück (64) aufweist, das an einer Sollbruchstelle (66) abbrechbar ist, und daß die Nadelabtrenneinrichtung (18, 19) eine im Bereich des zweiten Endes (15) des Schlitzes (12) angeordnete Klemmeinrichtung (18, 19) aufweist, in die das Endstück (64) der chirurgischen Nadel (60) in quer zu dem Schlitz (12) verlaufender Anordnung der chirurgischen Nadel (60) einklemmbar ist.

4. System nach Anspruch 1 oder 2, **dadurch gekennzeichnet, daß** die Nadelabtrenneinrichtung (70) eine Schneidzone (70) aufweist, die über eine im Bereich des zweiten Endes (15) des Schlitzes (12) angeordnete Öffnung (72) zugänglich ist.

5. System nach Anspruch 1 oder 2, **dadurch gekennzeichnet, daß** die Nadelabtrenneinrichtung (80) eine Schneideinrichtung (80, 82) aufweist.

6. System nach einem der Ansprüche 1 bis 5, **dadurch gekennzeichnet, daß** das Gehäuse (2) eine Wickeleinrichtung (36, 37) zur Aufnahme überschüssiger Leitungslänge der Herzleitung aufweist.

7. System nach Anspruch 6, **dadurch gekennzeichnet, daß** die Wickeleinrichtung (36, 37) mindestens zwei an der Außenseite des Gehäuses (2) vorgesehene Einkerbungen (36, 37) aufweist.

8. System nach einem der Ansprüche 1 bis 7, **dadurch gekennzeichnet, daß** mindestens ein Schnellkontakt (28, 29) eine Kontaktfeder (28, 29) aufweist.

9. System nach einem der Ansprüche 1 bis 8, **dadurch gekennzeichnet, daß** die Verbindungsvorrichtung (1) eine Zugentlastung (30) für den proximalen Endbereich (52) der temporären Herzleitung (50) aufweist.

10. System nach Anspruch 9, **dadurch gekennzeichnet, daß** die Zugentlastung (30) eine in einer Richtung einfedernde und in der entgegengesetzten Richtung sperrende Blattfeder (30) aufweist, die zum Angriff am proximalen Endbereich (52) der temporären Herzleitung (50) eingerichtet ist.

11. System nach einem der Ansprüche 1 bis 10, **dadurch gekennzeichnet, daß** die Herzleitung als monopolare Herzleitung mit einer distalen und einer proximalen Elektrode gestaltet ist.

12. System nach einem der Ansprüche 1 bis 10, **dadurch gekennzeichnet, daß** die Herzleitung (50) als bipolare Herzleitung mit zwei distalen und zwei proximalen Elektroden (56, 58) gestaltet ist.

13. System nach Anspruch 12, **dadurch gekennzeichnet, daß** die Herzleitung (50) zumindest in ihrem proximalen Endbereich (52) koaxial gestaltet ist.

14. System nach Anspruch 13, **dadurch gekennzeichnet, daß** die beiden proximalen Elektroden (56, 58) ringförmig sind und auf Abstand zueinander angeordnet sind.

15. System nach Anspruch 14, **dadurch gekennzeichnet, daß** die beiden ringförmigen Elektroden (56, 58) gleichen Außendurchmesser haben, der an den Außendurchmesser der äußeren Isolierung (55) im proximalen Bereich der Herzleitung (50) angepaßt ist.

16. Externe Verbindungsvorrichtung (1) für eine temporäre Herzleitung (50), die einen distalen Endbereich mit mindestens einer distalen Elektrode und einen proximalen Endbereich (52) mit mindestens einer proximalen Elektrode (56, 58) und eine abtrennbare, nicht als Elektrode eingerichtete chirurgische Nadel (60) aufweist, wobei eine jeweilige distale Elektrode elektrisch leitend mit einer jeweiligen proximalen Elektrode (56, 58) verbunden ist,
wobei die Verbindungsvorrichtung (1) ein Gehäuse (2) mit einer Eintrittsöffnung (10) und einer Nadelaustrittsöffnung (12) und eine Nadelabtrenneinrichtung (18, 19; 70; 80) aufweist, wobei im Innenraum des Gehäuses (2) jeweiligen proximalen Elektroden (56, 58) der temporären Herzleitung (50) zugeordnete Schnellkontakte (28, 29) angeordnet sind, die über mindestens ein Kabel (40, 42) mit einem externen Herzschrittmacher verbindbar sind,
**dadurch gekennzeichnet, daß** die Nadelaustrittsöffnung als Schlitz (12) mit einem ersten Ende (14) und einem zweiten Ende (15) in einer Wand (8) des Gehäuses (2) geformt ist, der an seinem ersten Ende (14) eine Öffnung hat und dort in die Eintrittsöffnung (10) übergeht und der entlang eines im Innenraum des Gehäuses (2) ausgebildeten Kanals (20) zum Aufnehmen eines Teils des proximalen Endbereichs (52) der temporären Herzleitung (50) verläuft und mit dem Kanal (20) in Verbindung steht.

17. Verbindungsvorrichtung nach Anspruch 16, **gekennzeichnet durch** die die Verbindungsvorrichtung (1) betreffenden Merkmale eines der Ansprüche 2 bis 15.

## Revendications

1. Système muni d'une dérivation cardiaque temporaire (50) et d'un dispositif de connexion externe (1) pour cette dernière,
- dans lequel la dérivation cardiaque temporaire (50) comporte une région d'extrémité distale avec au moins une électrode distale et une région d'extrémité proximale (52) avec au moins une électrode proximale (56, 58) et une aiguille chirurgicale cassable (60), ne servant pas d'électrode, une électrode distale respective étant connectée de manière électriquement conductrice à une électrode proximale respective (56, 58), et
- dans lequel le dispositif de connexion (1) comporte un logement (2) doté d'une ouverture d'entrée (10) et d'une ouverture de sortie d'aiguille (12) et comporte un dispositif de rupture d'aiguille (18, 19 ; 70 ; 80), à l'intérieur du logement (2) étant agencés des contacts à action rapide (28, 29), attribués à des électrodes proximales respectives (56, 58) de la connexion cardiaque temporaire (50), qui peut être connectée via au moins un câble (40, 42) à un stimulateur cardiaque externe,
**caractérisé en ce que** l'ouverture de sortie d'aiguille se présente sous la forme d'une fente (12) avec une première extrémité (14) et une seconde extrémité (15) dans une paroi (8) du logement (2), laquelle fente possède une ouverture au niveau de sa première extrémité (14) et là passe dans l'ouverture d'entrée (10) et laquelle fente s'étend le long d'un conduit (20) formé à l'intérieur du logement (2) pour loger une partie de la région d'extrémité proximale (52) de la dérivation cardiaque temporaire (50) et communique avec ledit conduit (20).

2. Système selon la revendication 1, **caractérisé en ce que** le logement (2) comporte une base (5), une paroi latérale (6) et une partie supérieure (8), l'ouverture d'entrée (10) étant formée dans la paroi latérale (6) et la fente (12) dans la base (5) ou dans la partie supérieure (8).

3. Système selon la revendication 1 ou 2, **caractérisé en ce que** l'aiguille chirurgicale (60) comporte un tenon (64), agencé à l'opposé de sa pointe (62), lequel peut être cassé au niveau d'un point de fracture déterminé (66), et **en ce que** le dispositif de rupture d'aiguille (18, 19) comporte un dispositif de serrage (18, 19), agencé dans la région de la seconde extrémité (15) de la fente (12), dans lequel le tenon (64) de l'aiguille chirurgicale (60) peut être serré dans un agencement de l'aiguille chirurgicale (60) s'étendant de façon transversale à la fente (12).

4. Système selon la revendication 1 ou 2, **caractérisé en ce que** le dispositif de rupture d'aiguille (70) comporte une zone de découpe (70) qui est accessible vie une ouverture (72) agencée dans la région de la seconde extrémité (15) de la fente (12).

5. Système selon la revendication 1 ou 2, **caractérisé en ce que** le dispositif de rupture d'aiguille (80) comporte un dispositif de découpe (80, 82).

6. Système selon l'une des revendications 1 à 5, **caractérisé en ce que** le logement (2) comporte un dispositif d'enroulement (36, 37) destiné à prendre le surplus de longueur de ligne de la dérivation cardiaque.

7. Système selon la revendication 6, **caractérisé en ce que** le dispositif d'enroulement (36, 37) comporte au moins deux renfoncements (36, 37) disposés sur l'extérieur du logement (2).

8. Système selon l'une des revendications 1 à 7, **caractérisé en ce qu'**au moins un contact à action rapide (28, 29) comporte un ressort de contact (28, 29).

9. Système selon l'une des revendications 1 à 8, **caractérisé en ce que** le dispositif de connexion (1) comporte un moyen de soulagement de tension (30) pour la région d'extrémité proximale (52) de la dérivation cardiaque temporaire (50).

10. Système selon la revendication 9, **caractérisé en ce que** le moyen de soulagement de tension (30) comporte un ressort à lames (30), fléchissant dans une direction et se bloquant dans la direction opposée, lequel est établi pour s'engager au niveau de la région d'extrémité proximale (52) de la dérivation cardiaque temporaire (50).

11. Système selon l'une des revendications 1 à 10, **caractérisé en ce que** la dérivation cardiaque est configurée en tant que dérivation cardiaque monopolaire avec une électrode distale et une électrode proximale.

12. Système selon l'une des revendications 1 à 10, **caractérisé en ce que** la dérivation cardiaque (50) est configurée en tant que dérivation cardiaque bipolaire avec deux électrodes distales et deux électrodes proximales (56, 58).

13. Système selon la revendication 12, **caractérisé en ce que** la dérivation cardiaque (50) est coaxiale au moins dans sa région d'extrémité proximale.

14. Système selon la revendication 13, **caractérisé en ce que** les deux électrodes proximales (56, 58) sont annulaires et sont espacées l'une de l'autre.

15. Système selon la revendication 14, **caractérisé en ce que** les deux électrodes annulaires (56, 58) ont le même diamètre extérieur, lequel correspond au diamètre extérieur de l'isolation extérieure (55) dans la région proximale de la dérivation cardiaque (50).

16. Dispositif de connexion externe (1) destiné à une dérivation cardiaque temporaire (50), qui comporte une région d'extrémité distale avec au moins une électrode distale et une région d'extrémité proximale (52) avec au moins une électrode proximale (56, 58) et une aiguille chirurgicale cassable (60), ne servant pas d'électrode, une électrode distale respective étant connectée de manière électriquement conductrice à une électrode proximale respective (56, 58),
dans lequel le dispositif de connexion (1) comporte un logement (2) doté d'une ouverture d'entrée (10) et d'une ouverture de sortie d'aiguille (12) et un dispositif de rupture d'aiguille (18, 19 ; 70 ; 80), à l'intérieur du logement (2) étant agencés des contacts à action rapide (28, 29), attribués à des électrodes proximales respectives (56, 58) de la connexion cardiaque temporaire (50), qui peut être connectée via au moins un câble (40, 42) à un stimulateur cardiaque externe,
**caractérisé en ce que** l'ouverture de sortie d'aiguille se présente sous la forme d'une fente (12) avec une première extrémité (14) et une seconde extrémité (15) dans une paroi (8) du logement (2), laquelle fente possède une ouverture au niveau de sa première extrémité (14) et là passe dans l'ouverture d'entrée (10) et laquelle fente s'étend le long d'un conduit (20) formé à l'intérieur du logement (2) pour loger une partie de la région d'extrémité proximale (52) de la dérivation cardiaque temporaire (50) et communique avec ledit conduit (20).

17. Dispositif de connexion selon la revendication 16, **caractérisé par** les caractéristiques de l'une des revendications 2 à 15 concernant le dispositif de connexion (1).
